# EUROPÄISCHE PATENTSCHRIFT

(11) **EP 4 493 082 B1**
(45) Veröffentlichungstag und Bekanntmachung des Hinweises auf die Patenterteilung: **22.07.2026**
(21) Anmeldenummer: 23708690.5
(22) Anmeldetag: 23.02.2023
(51) Int. Cl.: A61B 17/34

(54) **ZIELGERÄT ZUR GEZIELTEN FÜHRUNG EINER HOHLNADEL BEI EINEM ENDOSKOPISCH-CHIRURGISCHEN EINGRIFF**
TARGET DEVICE FOR THE TARGETED GUIDANCE OF A HOLLOW NEEDLE DURING AN ENDOSCOPIC SURGICAL PROCEDURE
DISPOSITIF CIBLE POUR LE GUIDAGE CIBLÉ D'UNE AIGUILLE CREUSE PENDANT UNE INTERVENTION CHIRURGICALE ENDOSCOPIQUE

(30) Priorität: 14.03.2022 DE 102022202486
(43) Veröffentlichungstag der Anmeldung: 22.01.2025
(73) Patentinhaber: Richard Wolf GmbH, 75438 Knittlingen (DE)
(72) Erfinder: FREY, Sebastian, 68753 Waghäusel (DE); MAUL, Silas, 75180 Pforzheim (DE); GÖTHEL, Dirk, 75057 Kürnbach (DE); POTHOF, Frederick, 76646 Bruchsal (DE)
(74) Vertreter: Patentanwälte Hemmer Lindfeld Frese Partnerschaft mbB
(86) Internationale Anmeldenummer: PCT/DE2023/200037
(87) Internationale Veröffentlichungsnummer: WO 2023/174493

(56) Entgegenhaltungen:
- WO-A1-2024/021613
- WO-A1-2024/138212
- US-A1- 2010 241 106
- US-A1- 2010 324 560
- US-A1- 2012 330 159
- US-A1- 2014 358 087
- US-A1- 2022 287 732
- US-A1- 2024 350 154
- US-A1- 2025 195 094
- US-B1- 11 259 880

## Beschreibung

Die vorliegende Offenbarung betrifft ein Zielgerät zur gezielten Führung einer Hohlnadel bei einem endoskopisch-chirurgischen Eingriff sowie ein Endoskopie-System mit einem solchen Zielgerät, insbesondere zur minimal-invasiven Wirbelsäulenchirurgie.

Es gibt bereits Endoskopie-Systeme zur minimal-invasiven Wirbelsäulen-Endoskopie. Die US 2014/358087 A1 ist auf ein Gerät zur Bereitstellung von mehreren Zugangsöffnungen in Blutgefäße gerichtet. Die US 2012/330159 A1 beschreibt ein Nadelführungsgerät zum Einstechen einer Nadel in subkutanes Gewebe. Die US 2010/241106 A1 beschreibt ein Führungsgerät mit Markierungen, um ein medizinisches Gerät zielgerichtet zu platzieren. Die US 2010/324560 A1 beschreibt ein Führungsgerät für ein Bohrinstrument in der Wirbelsäulenchirurgie. Die US 11,259,880 B1 beschreibt ein Verfahren und ein System zur Trajektorenidentifizierung in der Wirbelsäulenchirurgie. Die EP 2 135 542 B1 beschreibt beispielweise eine für die minimal-invasive endoskopische Wirbelsäulen-Endoskopie bestimmte Endoskopie-Einheit in Form eines Endoskop- und Schaftsystems mit einem Zugangstubus und einem in den Zugangstubus einsetzbaren Endoskop. Typischerweise werden damit Dekompressionen an der Hals-, Brust- oder Lendenwirbelsäule durchgeführt, beispielsweise bei Bandscheibenvorfällen, spinalen Zysten oder Spinalkanalstenosen. Die Endoskopie-Längsachse kann dabei beispielsweise transforaminal, extraforaminal oder Interlaminär verlaufen.

Ein Problem an der bekannten Lösung ist, dass der Zugangsschaft nur begrenzt Platz bietet und einen Zugang zwischen die Wirbelkörper nur aus einer Richtung bietet. Für bestimmte chirurgische Eingriffe ist es allerdings vorteilhaft oder sogar notwendig, dass zusätzlich zu dem mittels der Endoskopie-Einheit geschaffenen Zugang in den Wirbelkörperzwischenraum ein zweiter Zugang in den Wirbelkörperzwischenraum aus einer anderen Richtung geschaffen werden muss.

Der zweite Zugang kann dabei mittels einer Hohlnadel aus einem anderen Winkel gestochen werden, um einen darin befindlichen Führungsdraht zu platzieren. Nach erfolgter Platzierung des Führungsdrahts kann die Hohlnadel proximalwärts vom Führungsdraht abgezogen werden und eine starre oder flexible Arbeitshülse über den Führungsdraht distalwärts eingedrückt werden. Es können auch sukzessiv mehrere Arbeitshülsen übereinander geschoben werden, um einen erforderlichen Zugangsquerschnitt zu erzielen. Sobald der erforderliche Zugangsquerschnitt erreicht ist, kann der Führungsdraht sowie ggf. innere Arbeitshülsen proximalwärts aus der äußersten Arbeitshülse herausgezogen werden.

Problematisch ist allerdings die korrekte Ausrichtung und Platzierung der Hohlnadel, um den gewünschten Zielbereich genau zu treffen. Bisher wird dies durch sehr erfahrenes chirurgisch geschultes Personal unter Röntgen- und/oder Ultraschall-Kontrolle durchgeführt. Es besteht allerdings immer ein Risiko, dass der Einstich der Hohlnadel beim ersten Versuch nicht optimal gesetzt wird und ggf. in einem oder mehr weiteren Versuchen neu gesetzt werden muss.

Daraus ergibt sich die Aufgabe, das Einstechen der Hohlnadel zu vereinfachen und damit das Risiko von Fehlstichen zu reduzieren.

Gemäß einem ersten Aspekt der vorliegenden Offenbarung wird zur Lösung dieses Problems ein Zielgerät zur gezielten Führung einer Hohlnadel einer bestimmten Länge bei einem endoskopisch-chirurgischen Eingriff bereitgestellt, wobei das Zielgerät an einer sich im Wesentlichen in einer Endoskopie-Längsachse erstreckenden und einen Sichtbereich definierenden Endoskopie-Einheit befestigt oder befestigbar ist, wobei das Zielgerät eine Mehrzahl von Hohlnadelführungen definiert, wobei sich jede der Hohlnadelführungen jeweils entlang eines anderen Polwinkels gegenüber der Endoskop-Längsachse auf den Sichtbereich zu erstreckt, wobei jede der Hohlnadelführungen über ihre gesamte Länge zu einer ihrer zwei azimutalen Seiten hin offen ist.

Der Sichtbereich kann als Schärfeebene im distalseitig vor der Endoskopie-Einheit liegenden Objektraum definiert sein, der mittels eines Endoskops der Endoskopie-Einheit beobachtbar ist. Für das Verständnis der vorliegenden Offenbarung ist es sinnvoll, ein lokales Kugelkoordinaten-System des Endoskopie-Systems bestehend aus Endoskopie-Einheit und daran montiertem Zielgerät zu definieren. Der Mittelpunkt des Kugelkoordinaten-Systems kann dabei als Mittelpunkt des distalseitig vor der Endoskopie-Einheit angeordneten Sichtbereichs definiert sein. Die Endoskopie-Längsachse kann entsprechend als Polachse z definiert sein. Bei einem beispielsweise interlaminären chirurgischen Eingriff an der Wirbelsäule eines auf dem Bauch liegenden Patienten verläuft die Endoskopie-Längsachse, also die Polachse, vorzugsweise vertikal. Die Hohlnadelführungen können meridional aufgereiht angeordnet sein und erstrecken sich jeweils in einem für jede Hohlnadelführung zugehörigen Polwinkel radial auf den Sichtbereich zu. Vorzugsweise erstrecken sich die Hohlnadelführungen möglichst genau auf den Mittelpunkt oder einen Innenbereich des Sichtbereichs zu. Es ist allerdings prinzipiell ausreichend, wenn eine in der jeweiligen Hohlnadelführung geführte Hohlnadel den Sichtbereich so genau trifft, dass die distale Spitze der Hohlnadel mit einem Endoskop der Endoskopie-Einheit beobachtet werden kann.

Das Zielgerät legt also durch die Hohlnadelführungen eindeutig fest, dass die distale Spitze einer Hohlnadel, unabhängig davon, welche der wahlweise zur Verfügung stehenden Hohlnadelführungen gewählt wird, immer im Sichtbereich des Endoskops landet und dort unter Sicht mittels des Endoskops final positioniert werden kann. Es kann daher eine Röntgen- und/oder Ultraschall-Kontrolle zur Platzierung der Hohlnadel entfallen, was den chirurgischen Eingriff wesentlich vereinfacht. Das Risiko eines Fehlstichs wird durch die Hohlnadelführungen reduziert, da neben der Auswahl der Hohlnadelführung nur die Einstichtiefe der Hohlnadel und die azimutale Drehstellung des Zielgeräts als Freiheitsgrade verbleiben. Die azimutale Drehstellung des Zielgeräts verläuft typischerweise quer zur (bei auf dem Bauch liegendem Patienten horizontal verlaufenden) Wirbelsäulen-Längsachse, sodass wahlweise schräg von links oder schräg von rechts zwischen die Wirbelkörper eingestochen wird. Vorzugsweise bestimmt das Zielgerät durch einen definierten Anschlag an jeder Hohlnadelführung auch die Einstichtiefe der Hohlnadel mit definierter Länge, sodass als einziger Freiheitsgrad die Auswahl der Hohlnadelführung verbleibt, wenn die azimutale Drehstellung des Zielgeräts gewählt ist. Die Auswahl der Hohlnadelführung bestimmt den Polwinkel des Einstichs gegenüber der Endoskop-Längsachse.

Die Hohlnadel kann nach finaler Platzierung des in der Hohlnadel befindlichen Führungsdrahts proximalwärts aus dem Patienten und dem Zielgerät herausgezogen werden, wobei der Führungsdraht positioniert im Patienten verbleibt bzw. dabei von der Bedienperson in einem proximal aus der Hohlnadel herausragenden Führungsdrahtabschnitt manuell festgehalten wird. Der Führungsdraht ist vorzugsweise sehr viel länger als die Hohlnadel. Dadurch, dass jede der Hohlnadelführungen über ihre gesamte Länge zu einer ihrer zwei azimutalen Seiten hin offen ist, kann der Führungsdraht einfach seitlich aus dem Zielgerät genommen werden und/oder das Zielgerät azimutal so verschwenkt werden, dass der Führungsdraht seitlich aus der Hohlnadelführung rutscht. Der relativ lange Führungsdraht muss also nicht mühsam unter starker Krümmung distalwärts aus der Hohlnadelführung gezogen werden. Das Merkmal, dass jede der Hohlnadelführungen über ihre gesamte Länge zu einer ihrer zwei azimutalen Seiten hin offen ist, ist also so zu verstehen, dass ein in einer Hohlnadelführung befindlicher Führungsdraht, nachdem die Hohlnadel proximalwärts aus der Hohlnadelführung gezogen wurde, seitlich aus der Hohlnadelführung herausgenommen werden kann. Um eine Führung der Hohlnadel bereitzustellen, sind die Hohlnadelführungen nicht so weit offen, dass die Hohlnadel selbst seitlich herausgenommen werden kann. Die Hohlnadel kann nur proximalwärts aus den Hohlnadelführungen gezogen werden. Vorzugsweise kann jede der Hohlnadelführungen einen Innendurchmesser zur axialen Aufnahme der Hohlnadel haben und auf ihrer offenen azimutalen Seite einen Öffnungsschlitz mit einer lichten Schlitzbreite aufweisen, wobei die lichte Schlitzbreite kleiner ist als der Innendurchmesser der Hohlnadelführungen. Die lichte Schlitzbreite ist also vorzugsweise kleiner als der Außendurchmesser der Hohlnadel, aber größer als der Außendurchmesser eines in der Hohlnadel befindlichen Führungsdrahts. Vorzugsweise ist die lichte Schlitzbreite kleiner als der Außendurchmesser der Hohlnadel, aber mindestens so groß wie der Innendurchmesser der Hohlnadel.

Nach erfolgter Platzierung des Führungsdrahts mittels der Hohlnadel und nach proximalwärtigem Herausziehen der Hohlnadel aus der gewählten Hohlnadelführung wird das Zielgerät zunächst nicht mehr benötigt und kann azimutal weggeschwenkt werden und/oder von der Endoskopie-Einheit abmontiert werden. Sodann kann nach bekannter Art und Weise eine starre oder flexible Arbeitshülse über den Führungsdraht distalwärts in den Patienten eingedrückt werden. Es können dann auch sukzessiv mehrere Arbeitshülsen übereinander geschoben werden, um einen erforderlichen Zugangsquerschnitt zu erzielen. Sobald der erforderliche Zugangsquerschnitt erreicht ist, kann der Führungsdraht sowie ggf. innere Arbeitshülsen proximalwärts aus der äußersten Arbeitshülse herausgezogen werden.

Optional kann jede Hohlnadelführung jeweils ein proximales Führungsende und ein distales Führungsende aufweisen, wobei die proximalen Führungsenden der Hohlnadelführungen im Wesentlichen den gleichen Abstand vom Sichtbereich haben. Damit steht für die Hohlnadel mit definierter Länge jede Hohlnadelführung zur Verfügung. Alternativ dazu kann es für jede Hohlnadelführung oder Gruppen von Hohlnadelführungen eine zugehörige Länge der darin zu verwendenden Hohlnadeln geben. Bevorzugt ist jedoch, dass jede Hohlnadelführung mit einer Hohlnadel mit definierter Länge verwendbar konzipiert ist, d.h. die proximalen Führungsenden der Hohlnadelführungen sind auf einer virtuellen Kugelfläche um den Sichtbereich angeordnet, wobei die virtuelle Kugelfläche einen Radius hat, welcher der Länge der Hohlnadel entspricht. Das Zielgerät ist also vorzugsweise für eine Hohlnadellänge angepasst konzipiert.

Optional kann für jedes beliebig aus den Hohlnadelführungen gewählte Paar gelten, dass sich das distale Führungsende einer ersten Hohlnadelführung des Paars entlang eines ersten Polwinkels erstreckt und einen ersten Abstand vom Sichtbereich hat und sich das distale Führungsende einer zweiten Hohlnadelführung des Paars entlang eines zweiten Polwinkels erstreckt und einen zweiten Abstand vom Sichtbereich hat, wobei der erste Polwinkel kleiner als der zweite Polwinkel ist und der erste Abstand kleiner als der zweite Abstand ist. Die Hohlnadelführungen mit größerem Abstand zur Endoskop-Längsachse können kürzer ausgestaltet sein als Hohlnadelführungen mit geringerem Abstand zur Endoskop-Längsachse. Dies ist besonders sinnvoll, wenn die Endoskop-Längsachse bei einem beispielsweise interlaminären chirurgischen Eingriff vertikal von oben auf eine Wirbelsäule eines auf dem Bauch liegenden Patienten ausgerichtet ist, um ein durch das Gewicht des Zielgeräts und die Hohlnadel ausgeübtes Kippmoment möglichst gering zu halten. Das Gewicht des Zielgeräts ist daher insbesondere in den von der Endoskop-Längsachse weit entfernten Bereichen möglichst gering zu halten.

Optional können sich die proximalen Führungsenden der Hohlnadelführungen proximalwärts trichterartig aufweiten und jeweils einen definierten Anschlag für die Hohlnadel bilden. Dies ist besonders sinnvoll, um einer Bedienperson das Einführen der Hohlnadel in das Zielgerät zu erleichtern und eine visuelle Überprüfung der korrekt vollständig eingeführten Hohlnadel bereitzustellen.

Optional können benachbarte Hohlnadelführungen jeweils am proximalen Führungsende und am distalen Führungsende miteinander verbunden sein. Eine Verbindung dazwischen ist nicht notwendig und kann durch Weglassen das Gewicht des Zielgeräts reduzieren.

Optional können die Hohlnadelführungen abwechselnd zu unterschiedlichen ihrer zwei azimutalen Seiten hin offen sein. Das heißt, jede zweite Hohlnadelführung ist nach rechts offen und die dazwischenliegenden Hohlnadelführungen sind nach links offen oder umgekehrt. Dies hat Vorteile in der Herstellung, da sich das Zielgerät dann einfacher einstückig ohne sich nach rechts oder links verziehende Materialspannungen herstellen lässt.

Optional kann das Zielgerät ferner ein Befestigungselement zum werkzeuglos lösbaren Befestigen an der Endoskopie-Einheit aufweisen. Vorzugsweise erlaubt das Befestigungselement eine sichere, feste Verbindung, welche die translatorische Positionierung und rotatorische Ausrichtung des Zielgeräts relativ zur Endoskopie-Einheit vollständig festlegt und darin sichert.

Optional kann das Befestigungselement dazu ausgestaltet sein, bestimmungsgemäß einen Zugangstubus der Endoskopie-Einheit zumindest teilweise zu umgreifen und daran angeklemmt zu werden. Ein Endoskop kann dann unabhängig vom Zielgerät in den Zugangstubus eingesteckt werden. Vorzugsweise weist das Zielgerät dazu proximal vom Befestigungselement einen Abstand zur Endoskop-Längsachse, damit ein Endoskop vollständig in den Zugangstubus eingesteckt werden kann.

Optional kann das Befestigungselement an einem distalen Endbereich des Zielgeräts angeordnet sein. Dadurch hat das Zielgerät einen ausreichend großen Abstand zum Sichtbereich, sodass die Hohlnadelführungen mit ausreichendem Abstand zueinander angeordnet sein können, um mit den zugehörigen Polwinkeln möglichst viele verschiedene Auswahlmöglichkeiten für den Einstichwinkel über einen möglichst großen Polwinkelbereich bereitzustellen.

Optional kann das Befestigungselement einen Schnellspannhebel oder Klemmhebel aufweisen. Vorzugsweise kann das Befestigungselement ein Formschlusselement aufweisen, welches dazu ausgestaltet ist, einen Formschluss mit einem an der Endoskopie-Einheit an einer definierten Position entlang der Endoskop-Längsachse vorgesehenen korrespondierenden Formschlusselement zu bilden. Durch den Formschluss des Formschlusselement an der definierten Position kann die translatorische Positionierung und rotatorische Ausrichtung des Zielgeräts relativ zur Endoskopie-Einheit eindeutig bestimmt sein, sodass eine fehlerhafte Montage des Zielgeräts an der Endoskopie-Einheit ausgeschlossen werden kann. Für die Montage des Zielgeräts an der Endoskopie-Einheit besteht also vorzugsweise kein Freiheitsgrad, von einer korrekten Montage des Zielgeräts an der Endoskopie-Einheit abzuweichen.

Optional können die Hohlnadelführungen durch ein einstückiges Führungselement gebildet sein. Dies ist sinnvoll, um die Teilevielfalt des Zielgeräts zu reduzieren und einen ungenauen Zusammenbau von Einzelteilen zu vermeiden. Die strengen Toleranzanforderungen für die Fertigung der Hohlnadelführungen sind leichter durch ein einstückiges Führungselement zu erreichen.

Optional können die Hohlnadelführungen als eine einstückige Einheit additiv gefertigt sein. Die Gesamtheit der Hohlnadelführungen kann dabei das einstückig gefertigte Führungselement bilden. Durch eine additive Fertigung, beispielsweise durch Selektives Laserschmelzen (LPBF, Laser Powder Bed Fusion), kann das Gesamtgewicht des Zielgeräts stark reduziert werden. Das einstückig gefertigte Führungselement, das die Hohlnadelführungen bildet, kann additiv aus einem leichten Metall, einer leichten Metalllegierung, und /oder einem leichten Kunststoff gefertigt sein. Das Material des Führungselement kann offen und/oder geschlossene Hohlräume aufweisen, um Gewicht zu reduzieren und die strukturelle Stabilität weitgehend aufrecht zu erhalten. Beispielsweise kann das Gesamtgewicht des Zielgeräts weniger als 100 Gramm, vorzugsweise 70 bis 80 Gramm, betragen. Die strukturelle Stabilität des Zielgeräts zeichnet allerdings sich in jedem Fall durch eine hohe Steifigkeit aus.

Erfindungsgemäß ist das Zielgerät zwischen den Hohlnadelführungen azimutal durchbrochen. Dies spart insbesondere Gewicht und Material.

Optional kann zumindest eine der Hohlnadelführungen einen distalen Führungsabschnitt und einen proximalen Führungsabschnitt aufweisen, wobei das Zielgerät zwischen dem distalen Führungsabschnitt und dem proximalen Führungsabschnitt zumindest einen freien Abschnitt aufweist, in dem die Hohlnadel nicht geführt ist. Auch dies kann Gewicht und Material sparen, da die Hohlnadel grundsätzlich nur an zwei in Längsrichtung möglichst weit beabstandeten Stellen geführt sein muss, um die räumliche Ausrichtung der Hohlnadel eindeutig festzulegen.

Optional kann für jedes beliebig aus den Hohlnadelführungen gewählte Paar gelten, dass sich eine erste Hohlnadelführung des Paars entlang eines ersten Polwinkels erstreckt und sich eine zweite Hohlnadelführung des Paars entlang eines zweiten Polwinkels erstreckt, wobei der freie Abschnitt der ersten Hohlnadelführung länger ist als der freie Abschnitt der zweiten Hohlnadelführung und der erste Polwinkel kleiner als der zweite Polwinkel ist. Optional kann für jedes beliebig aus den Hohlnadelführungen gewählte Paar gelten, dass sich eine erste Hohlnadelführung des Paars entlang eines ersten Polwinkels erstreckt und sich eine zweite Hohlnadelführung des Paars entlang eines zweiten Polwinkels erstreckt, wobei die Länge der ersten Hohlnadelführung größer ist als die Länge der zweiten Hohlnadelführung und der erste Polwinkel kleiner als der zweite Polwinkel ist. Die Hohlnadelführungen können also wie die einzelnen Röhrchen einer Panflöte unterschiedlich lang sein, wobei die Länge mit wachsendem Polwinkel abnimmt. Dies verringert das durch das Gewicht des Zielgeräts ausgeübte Kippmoment auf die Endoskopie-Einheit.

Optional kann das Zielgerät neben den Hohlnadelführungen offene und/oder geschlossene Hohlkammern aufweisen, um Gewicht und Material zu sparen.

Optional können die Hohlnadelführungen meridional aufgereiht angeordnet sein. Die azimutale Ausdehnung des Zielgeräts ist dadurch möglichst schmal. Der meridionale Abstand der Hohlnadelführungen untereinander ist so gering wie möglich, um Material und Gewicht zu sparen, und so groß wie nötig, um eine ausreichende Auswahl an Einstichwinkeln über einen möglichst großen Polwinkelbereich bereitstellen zu können.

Gemäß einem weiteren Aspekt der vorliegenden Offenbarung wird ein Endoskopie-System für einen endoskopisch-chirurgischen Eingriff bereitgestellt, wobei das Endoskopie-System aufweist:
- eine sich im Wesentlichen in einer Endoskopie-Längsachse erstreckende und einen Sichtbereich definierende Endoskopie-Einheit,
- mindestens eine Hohlnadel, und
- ein zuvor beschriebenes Zielgerät zur gezielten Führung der mindestens einen Hohlnadel entlang eines auswählbaren Polwinkels gegenüber der Endoskop-Längsachse auf den Sichtbereich zu, wobei das Zielgerät an der Endoskopie-Einheit befestigt oder befestigbar ist.

Optional kann die Endoskopie-Einheit einen sich im Wesentlichen in der Endoskopie-Längsachse erstreckenden Zugangstubus und ein von proximal in den Zugangstubus einsetzbares Endoskop aufweisen.

Optional kann das Zielgerät an einem proximalen Endabschnitt des Zugangstubus werkzeuglos lösbar befestigbar sein.

Optional kann die Hohlnadel mit einem innerhalb der Hohlnadel verlaufenden Führungsdraht ausgestattet sein, der proximalseitig aus der Hohlnadel herausragt und einen Außendurchmesser ausweist, der geringer ist als eine lichte Schlitzweite der azimutalen Öffnung jeder Hohlnadelführung des Zielgeräts.

Im Folgenden wird das hierin offenbarte System näher anhand der beiliegenden Figuren erläutert. Es zeigen:
- Fig. 1a-c: schematisch drei verschiedene chirurgische Zugangsmöglichkeiten in einen Zwischenwirbelraum;
- Fig. 2: eine Seitenansicht auf ein Ausführungsbeispiel eines hierin offenbarten Endoskopie-Systems;
- Fig. 3: eine Seitenansicht auf das in Fig. 2 gezeigte Endoskopie-System ohne Endoskop;
- Fig. 4: eine Seitenansicht auf ein Zielgerät des in Fig. 2 gezeigten Endoskopie-Systems;
- Fig. 5a,b: eine Draufsicht auf das in Fig. 4 gezeigte Zielgerät und einen Detail-Längsschnitt A-A;
- Fig. 6: eine Draufsicht auf das in Fig. 3 gezeigte Endoskopie-System ohne Endoskop mit proximalwärts aus der siebten Hohlnadelführung herausgezogener Hohlnadel;
- Fig. 7: eine perspektivische Detailansicht zur Darstellung, wie das in Fig. 4 gezeigte Zielgerät an einem distalen Endabschnitt eines Zugangstubus des Endoskopie-Systems moniert wird;
- Fig. 8a,b: Detail-Draufsichten vor und nach einer Montage des in Fig. 7 gezeigten Zielgeräts an einem distalen Endabschnitt eines Zugangstubus des Endoskopie-Systems; und
- Fig. 9: eine Seitenansicht auf ein weiteres Ausführungsbeispiel eines hierin offenbarten Endoskopie-Systems ohne Endoskop.

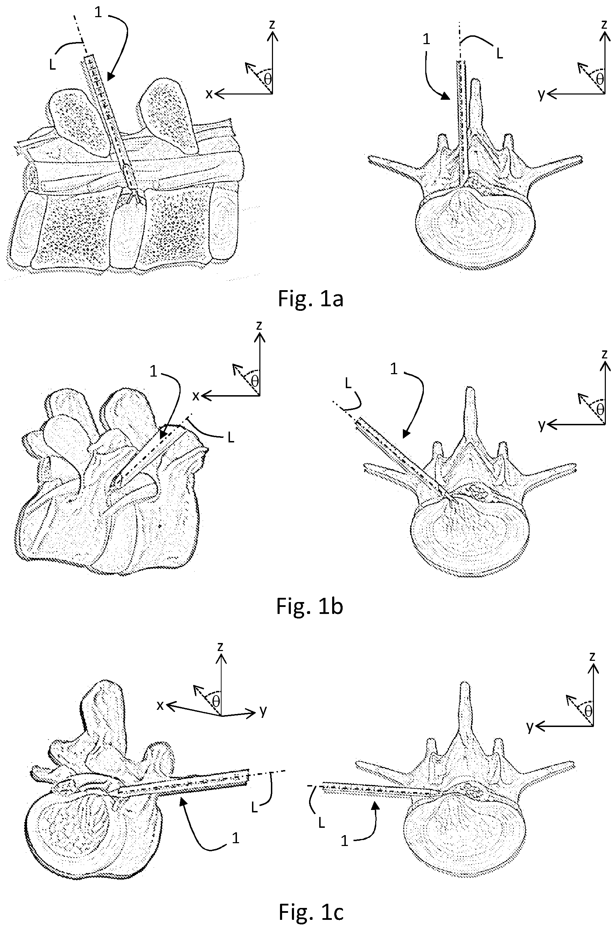

In Fig. 1a-c sind drei verschiedene chirurgische Zugangsmöglichkeiten in einen Zwischenwirbelraum gezeigt. Zu sehen ist jeweils links ein Längsschnitt oder perspektivischer Schnitt und rechts ein Querschnitt durch einen Wirbel einer Wirbelsäule eines auf dem Bauch liegenden Patienten. Zur Vereinfachung der Orientierung ist in allen Figuren ein rechtshändiges kartesisches Koordinatensystem gezeigt, bei dem die z-Achse vertikal, die x-Achse horizontal entlang der Wirbelsäule und die y-Achse horizontal lateral verläuft. Es ist zudem sinnvoll, die relativen Raumpositionen und -ausrichtungen mittels Kugelkoordinaten anzugeben, wobei der Polwinkel θ hier als Winkel zur z-Achse, ein Azimut-Winkel φ als Drehwinkel in der horizontalen xy-Ebene und r als Abstand zum Koordinatenursprung definiert ist.

In Fig. 1a ist ein interlaminarer Zugang gezeigt, durch den mittels einer Endoskopie-Einheit 1 in einen Zwischenwirbelraum chirurgisch eingegriffen wird. In den Figuren 1a-c ist nur ein distaler Endabschnitt der Endoskopie-Einheit 1 zu sehen. Die Endoskopie-Einheit 1 definiert eine Endoskopie-Längsachse L, die im Falle des in Fig. 1a gezeigten interlaminaren Zugangs relativ vertikal verläuft. Fig. 1b zeigt einen transforaminalen oder extraformaninalen Zugang von posterolateral und Fig. 1c einen transforaminalen oder extraformaninalen Zugang von lateral. Es sei hier angemerkt, dass der Patient so gelegt werden kann, dass die Endoskopie-Einheit 1 im Wesentlichen vertikal entlang der z-Achse verläuft. Bleibt der Patient in Bauchlage, ist es für die folgenden Figuren sinnvoll, das willkürlich definierte Koordinatensystem so zu definieren, dass die z-Achse entlang der Endoskopie-Längsachse L verläuft.

Für bestimmte chirurgische Eingriffe ist es nun vorteilhaft oder sogar notwendig, dass zusätzlich zu dem mittels der Endoskopie-Einheit 1 geschaffenen Zugang in den Wirbelkörperzwischenraum ein zweiter Zugang in den Wirbelkörperzwischenraum aus einer anderen Richtung geschaffen werden muss.

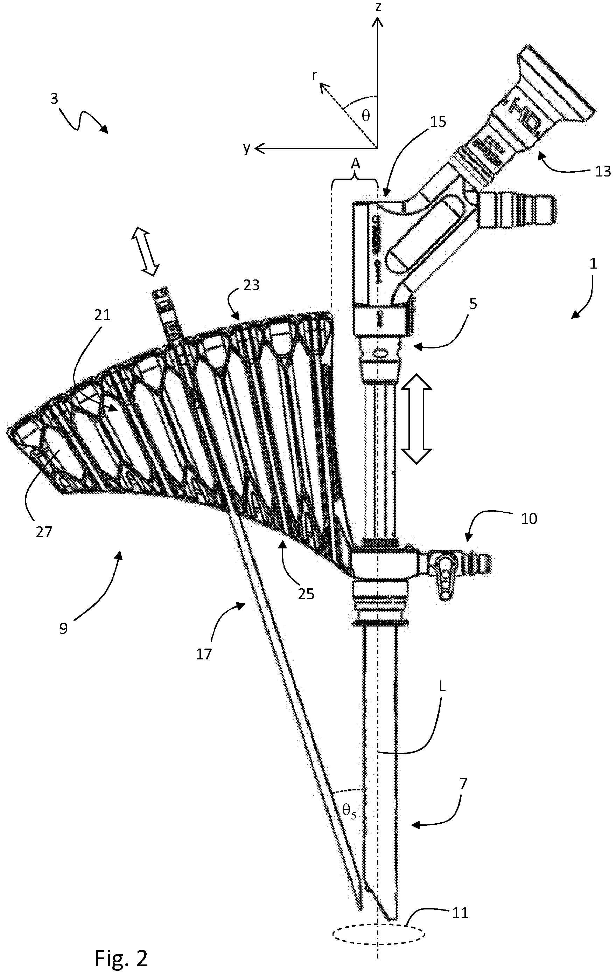

Fig. 2 bis 4 zeigen ein Ausführungsbeispiel eines erfindungsgemäßen Endoskopie-Systems 3, das die Schaffung eines zweiten Zugangs in den Wirbelkörperzwischenraum stark vereinfacht. Das Endoskopie-System 3 weist eine herkömmliche Endoskopie-Einheit 1, bestehend aus einem Endoskop 5 und einem Zugangstubus 7, sowie einem Zielgerät 9 und einer Hohlnadel 17 auf. Die Endoskopie-Einheit 1 definiert mit der Längsachse L des Zugangstubus 7 die Endoskopie-Längsachse L, die hier entlang der z-Achse verläuft. Das Endoskop 5 ist in den Zugangstubus 7 von proximal distalwärts einführbar. Der Zugangstubus 7 weist an seinem proximalen Endabschnitt einen Spülflüssigkeitseinlass und/oder -auslass 10 auf. Wenn das Endoskop 5 die maximal distale Position im Zugangstubus 7 eingenommen hat, befindet sich die distale Spitze des Endoskops 5 am distalen Ende des Zugangstubus 7 zum Einsehen eines distal vor der distalen Spitze des Endoskops 5 liegenden Sichtbereichs 11. Das Endoskop 5 weist vorzugsweise ein optisches System zum Beleuchten und Einsehen des Sichtbereichs 11 auf. Eine Bedienperson kann mittels eines Okulars 13 direkt mit dem Auge den Sichtbereich 11 einsehen oder (nicht gezeigt) mittels eines Adapters eine Kamera zur Bildgebung auf einem Bildschirm an das Okular 13 anschließen. Das Endoskop 5 weist vorzugsweise ferner einen Arbeitskanal mit einer proximalen Arbeitskanalöffnung 15 auf, durch welchen ein endoskopisches Werkzeug, wie etwa eine Resektionszange oder -schere, bis in den Sichtbereich 11 geschoben werden kann. Dadurch kann unter Sichtkontrolle der Zwischenwirbelraum chirurgisch operiert werden.

Der Sichtbereich 11 ist hier oval dargestellt und kann als quer zur Endoskop-Längsachse L verlaufende Schärfeebene im distalseitig vor der Endoskopie-Einheit 1 liegenden Objektraum definiert sein, der mittels des Endoskops 5 beobachtbar ist. Ein lokales Kugelkoordinaten-System des Endoskopie-Systems 3 kann daher so definiert werden, dass der Mittelpunkt des Kugelkoordinaten-Systems dabei als Mittelpunkt des distalseitig vor der Endoskopie-Einheit 1 angeordneten Sichtbereichs 11 ist. Die Endoskopie-Längsachse L ist entsprechend als Polachse z definiert. Bei einem beispielsweise interlaminären chirurgischen Eingriff an der Wirbelsäule eines auf dem Bauch liegenden Patienten verläuft die Endoskopie-Längsachse L, also die Polachse z, vorzugsweise vertikal.

Das erfindungsgemäße Zielgerät 9 dient der Vereinfachung der Schaffung eines zweiten Zugangs zum Sichtbereich 11 mittels der Hohlnadel 17. Das Zielgerät 9 weist ein Befestigungselement 19, hier mit einem Schnellspannhebel 33, zum werkzeuglos lösbaren Befestigen an einem proximalen Endabschnitt des Zugangstubus 7 auf. Das Zielgerät 9 ist somit während seines Einsatzes fest am Zugangstubus 7 arretiert. Sobald das Zielgerät nach erfolgreicher Platzierung der Hohlnadel 17 nicht mehr benötigt wird, kann es schnell werkzeuglos abmontiert werden, damit es bei der weiteren chirurgischen Operation nicht stört.

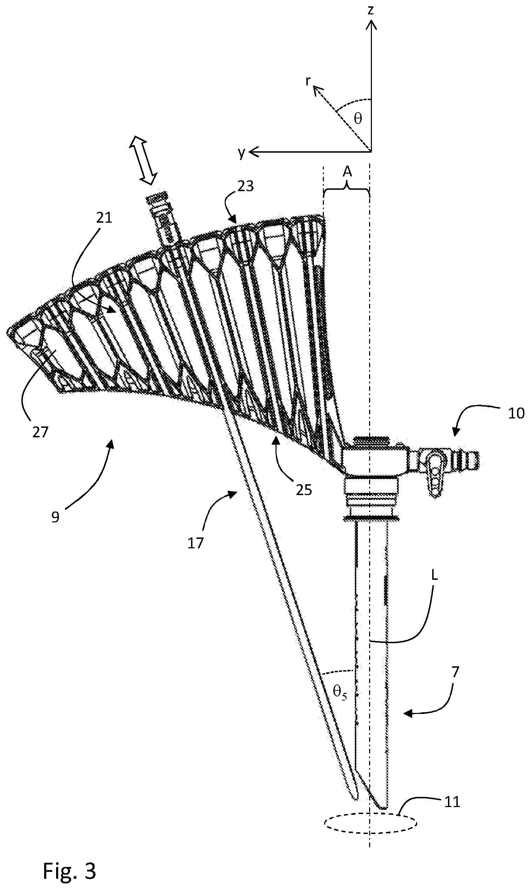

Das Zielgerät 9 definiert eine Mehrzahl (hier zehn) von fächerartig nebeneinander angeordneten Hohlnadelführungen 21, die sich jeweils entlang eines anderen Polwinkels θ gegenüber der Endoskop-Längsachse L auf den Sichtbereich 11 zu erstrecken. Die Hohlnadelführungen 21 sind meridional aufgereiht angeordnet und erstrecken sich jeweils in einem für jede Hohlnadelführung 21 zugehörigen Polwinkel θ radial auf den Sichtbereich 11 zu. In Fig. 2 und 3 ist die Hohlnadel 17 in die fünfte Hohlnadelführung 21 eingesteckt, welche sich unter dem Polwinkel θ₅ gegenüber der Endoskop-Längsachse L auf den Sichtbereich 11 zu erstreckt.

Jede Hohlnadelführung 21 weist jeweils ein proximales Führungsende 23 und ein distales Führungsende 25 auf, wobei die proximalen Führungsenden 23 der Hohlnadelführungen 21 im Wesentlichen den gleichen Abstand vom Sichtbereich 11 haben. Die proximalen Führungsenden 23 der Hohlnadelführungen 21 sind also in einem Kreis in der in Fig. 2 bis 4 gezeigten yz-Ebene um den Sichtbereich 11 angeordnet. Der Kreisradius ist durch die Länge der Hohlnadel 17 bestimmt. Damit steht für die Hohlnadel 17 mit definierter Länge jede Hohlnadelführung 21 zur Verfügung, d.h. das Zielgerät 9 ist für eine bestimmte Länge der Hohlnadel 17 angepasst konzipiert.

Die Hohlnadelführungen 21 mit größerem Abstand zur Endoskop-Längsachse L sind kürzer ausgestaltet als Hohlnadelführungen mit geringerem Abstand zur Endoskop-Längsachse L. Entsprechend ist der Abstand des distalen Führungsendes 25 vom Sichtbereich 11 größer bei Hohlnadelführungen 21 mit größerem Abstand zur Endoskop-Längsachse L als bei Hohlnadelführungen 21 mit kleinerem Abstand zur Endoskop-Längsachse L. Dies ist besonders für den Fall sinnvoll, wenn die Endoskop-Längsachse L bei einem beispielsweise interlaminären chirurgischen Eingriff vertikal von oben auf eine Wirbelsäule eines auf dem Bauch liegenden Patienten ausgerichtet ist, um ein durch das Gewicht des Zielgeräts 9 ausgeübtes Kippmoment möglichst gering zu halten. Das Gewicht des Zielgeräts 9 ist daher insbesondere in den von der Endoskop-Längsachse L weit entfernten Bereichen möglichst gering. Das Zielgerät 9 ist vorzugsweise, wie in Fig. 2 gezeigt, in Umfangsrichtung um die Endoskop-Längsachse L diametral zur Ausrichtung des Okulars 13 des Endoskops 5 ausgerichtet, d.h. zwischen diesen liegt ein Azimut-Winkel φ von ca. 180°. Dadurch heben sich die von Okular 13 und Zielgerät 9 verursachten Kippmomente zumindest teilweise gegeneinander auf.

Die proximalen Führungsenden 23 der Hohlnadelführungen 17 weiten sich proximalwärts trichterartig auf und bilden jeweils einen definierten Anschlag für die Hohlnadel 17. Dies ist besonders sinnvoll, um einer Bedienperson das Einführen der Hohlnadel 17 in das Zielgerät 9 zu erleichtern und eine visuelle Überprüfung der korrekt vollständig eingeführten Hohlnadel 17 bereitzustellen. Die Hohlnadel 17 kann damit nicht versehentlich zu tief eingestochen werden und die distale Spitze der Hohlnadel 17 landet immer im Sichtbereich 11, wo sie unter Sichtkontrolle final platziert werden kann.

Beim Zielgerät 9 sind benachbarte Hohlnadelführungen 21 jeweils am proximalen Führungsende 23 und am distalen Führungsende 25 miteinander verbunden. Eine Verbindung dazwischen ist nicht notwendig, sodass dort durch Weglassen von Material das Gewicht des Zielgeräts 9 reduziert ist. Das Zielgerät 9 weist also zwischen den Hohlnadelführungen 21 azimutale Durchbrechungen 27 auf.

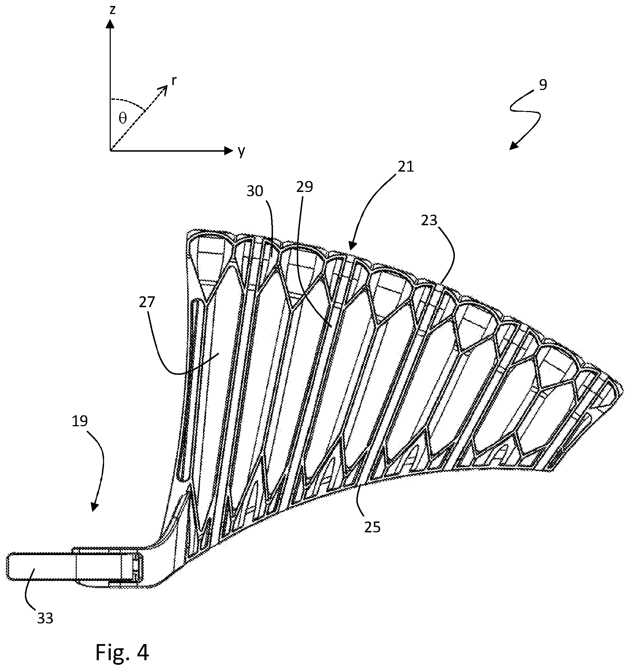

Erfindungsgemäß ist jede der Hohlnadelführungen 21 über ihre gesamte Länge zu einer ihrer zwei azimutalen Seiten hin offen. In dem in Fig. 2 bis 4 gezeigten Ausführungsbeispiel sind die Hohlnadelführungen 21 abwechselnd zu unterschiedlichen ihrer zwei azimutalen Seiten hin offen. Das heißt, jede zweite Hohlnadelführung ist nach rechts offen und die dazwischenliegenden Hohlnadelführungen sind nach links offen oder umgekehrt. In Fig. 2 und 3 sind von der Endoskop-Längsachse L aus die erste, dritte, fünfte, siebte und neunte Hohlnadelführung 21 zum Betrachter hin (negative x-Richtung) offen, wogegen die von der Endoskop-Längsachse L aus zweite, vierte, sechste, achte und zehnte Hohlnadelführung 21 vom Betrachter weg (negative x-Richtung) offen ist. In Fig. 4 ist das Zielgerät 9 von der anderen azimutalen Seite gezeigt. Die abwechselnd nach links und rechts offene Ausgestaltung hat Vorteile in der Herstellung, da sich das Zielgerät 9 dann einfacher einstückig herstellen lässt, ohne sich nach rechts oder links zu verziehen.

Das Merkmal, dass jede der Hohlnadelführungen 21 über ihre gesamte Länge zu einer ihrer zwei azimutalen Seiten hin offen ist, ist also so zu verstehen, dass ein in einer Hohlnadelführung 21 befindlicher Führungsdraht (nicht gezeigt), nachdem die Hohlnadel 17 proximalwärts aus der Hohlnadelführung 21 gezogen wurde, seitlich aus der Hohlnadelführung 21 herausgenommen werden kann. Um eine Führung der Hohlnadel 17 bereitzustellen, sind die Hohlnadelführungen 21 nicht so weit offen, dass die Hohlnadel 17 selbst seitlich herausgenommen werden kann. Die Hohlnadel 17 kann nur proximalwärts aus den Hohlnadelführungen 21 gezogen werden.

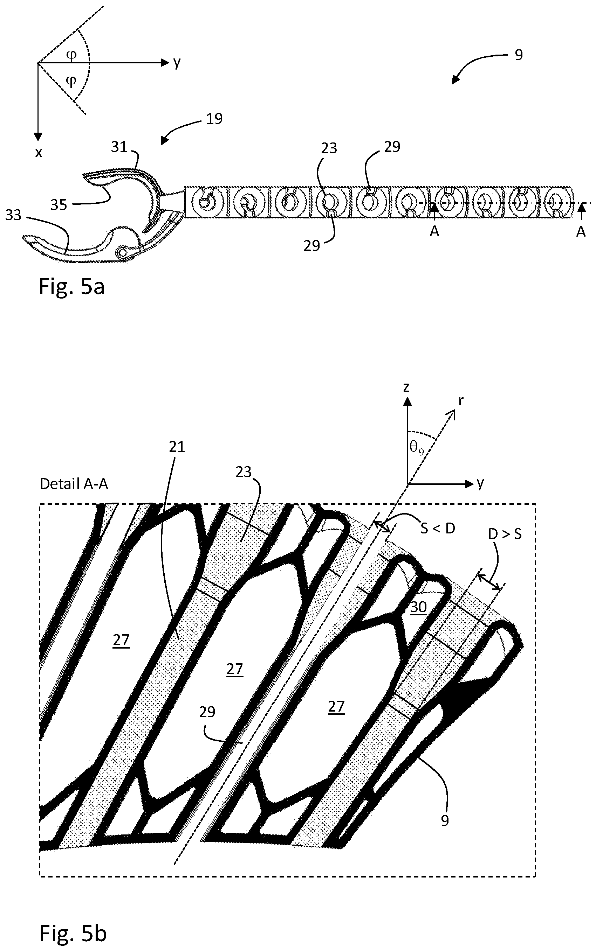

Wie in Fig. 5a,b genauer gezeigt, hat jede der Hohlnadelführungen 21 einen Innendurchmesser D zur axialen Aufnahme der Hohlnadel 17. Der Innendurchmesser D ist bei allen Hohlnadelführungen 21 gleich, sodass jede passgenau die Hohlnadel 17 mit entsprechendem Außendurchmesser aufnehmen kann. Auf ihrer offenen azimutalen Seite weist jede Hohlnadelführung 21 einen Öffnungsschlitz 29 mit einer lichten Schlitzbreite S auf, wobei die lichte Schlitzbreite S kleiner ist als der Innendurchmesser D der Hohlnadelführungen. Die lichte Schlitzbreite S ist also kleiner als der Außendurchmesser der Hohlnadel 17, aber größer als der Außendurchmesser eines in der Hohlnadel 17 befindlichen Führungsdrahts (nicht gezeigt). Vorzugsweise ist die lichte Schlitzbreite S kleiner als der Außendurchmesser der Hohlnadel 17, aber mindestens so groß wie der Innendurchmesser der Hohlnadel 17.

Zur Reduzierung der Teilevielfalt, der Kosten und des Gewichts des Zielgeräts 9 sind die Hohlnadelführungen 21 durch ein einstückiges Führungselement gebildet. Die strengen Toleranzanforderungen für die Fertigung der Hohlnadelführungen 21 sind zudem leichter durch ein einstückiges Führungselement zu erreichen. Das einstückige Führungselement ist hier additiv gefertigt. Die Gesamtheit der Hohlnadelführungen 21 bildet dabei das einstückig gefertigte Führungselement. Durch eine additive Fertigung, beispielsweise durch Selektives Laserschmelzen (LPBF, Laser Powder Bed Fusion), wird das Gesamtgewicht des Zielgeräts 9 stark reduziert. Beispielsweise kann das Gesamtgewicht des Zielgeräts 9 weniger als 100 Gramm, vorzugsweise 70 bis 80 Gramm, betragen. Die strukturelle Stabilität des Zielgeräts 9 zeichnet allerdings sich in jedem Fall durch eine hohe Steifigkeit aus. Das Zielgerät 9 kann neben den Hohlnadelführungen 21 offene und/oder geschlossene Hohlkammern 30 aufweisen, um Gewicht und Material zu sparen.

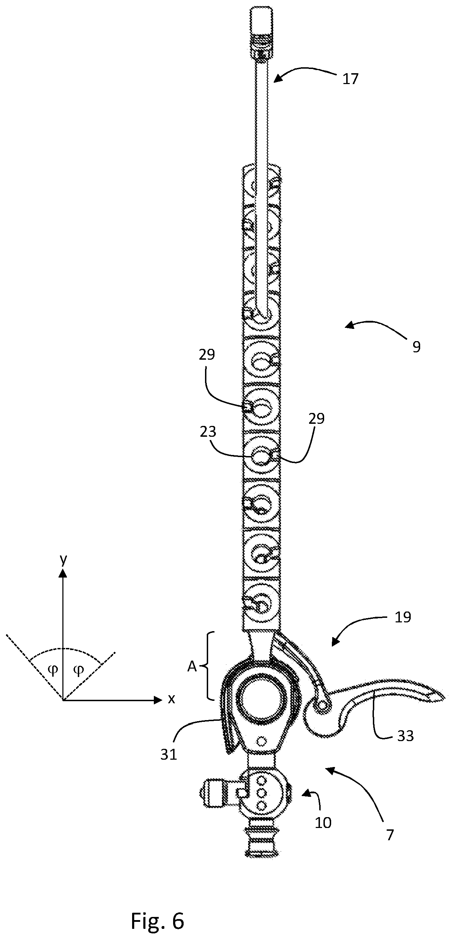

In der in Fig. 6 gezeigten Draufsicht ist die Hohlnadel 17 gerade vor dem Einführen in die von der Endoskopie-Längsachse L aus siebte Hohlnadelführung 21 gezeigt. Das Befestigungselement 19 ist dazu ausgestaltet, bestimmungsgemäß den Zugangstubus 7 der Endoskopie-Einheit 1 zumindest teilweise zu umgreifen und daran angeklemmt zu werden. Das Endoskop 5 kann dann unabhängig vom arretierten Zielgerät 9 in den Zugangstubus 7 eingesteckt werden. Das Zielgerät 7 weist dazu proximal vom Befestigungselement 19 einen Abstand A zur Endoskop-Längsachse L auf, damit das Endoskop 5 vollständig in den Zugangstubus 7 eingesteckt werden kann.

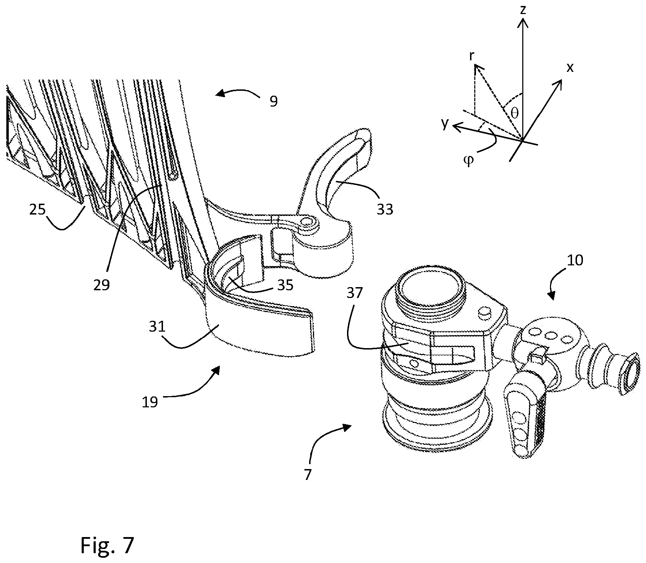

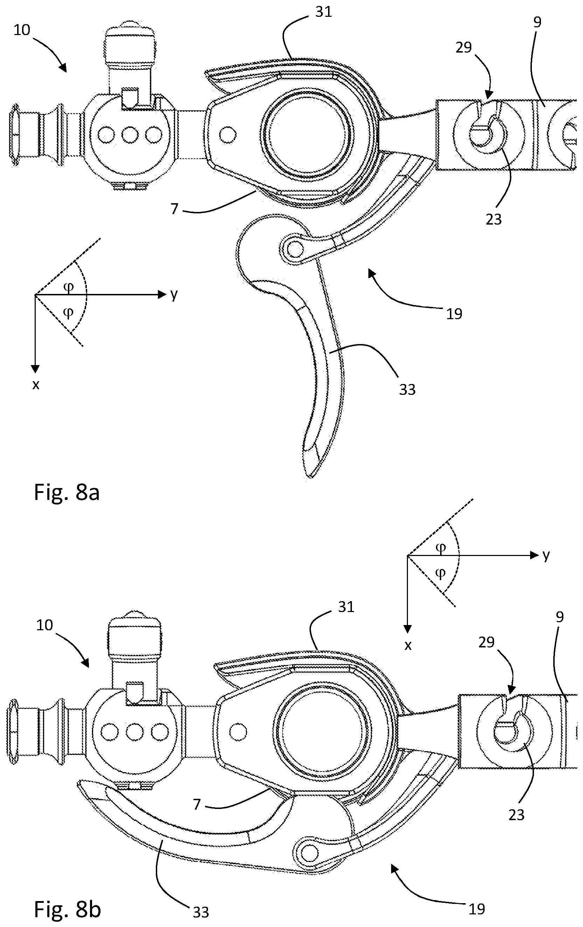

Das in Fig. 7 und Fig. 8a,b genauer gezeigte Befestigungselement 19 weist hier azimutal gegenüberliegend von einem den proximalen Endabschnitt des Zugangstubus 7 umgreifenden Griffhaken 31 des Zielgeräts 9 einen Schnellspannhebel 33 auf. Der Griffhaken 31 weist hier ein an den proximalen Endabschnitt des Zugangstubus 7 angepasstes Formschlusselement 35 in Form eines innenseitig in Umfangsrichtung verlaufenden Stegs auf, um einen Formschluss mit einem korrespondierend geformten Formschlusselement 37 in Form einer außenseitig in Umfangsrichtung verlaufenden Nut am Zugangstubus 7 zu bilden. Durch den Formschluss der Formschlusselement3 35, 37 miteinander an der definierten Position ist die translatorische Positionierung und rotatorische Ausrichtung des Zielgeräts relativ zur Endoskopie-Einheit eindeutig bestimmt, sodass eine fehlerhafte Montage des Zielgeräts 9 an der Endoskopie-Einheit 1 ausgeschlossen werden kann. Für die Montage des Zielgeräts 9 an der Endoskopie-Einheit 1 besteht also kein Freiheitsgrad, von einer korrekten Montage des Zielgeräts 9 an der Endoskopie-Einheit 1 abzuweichen.

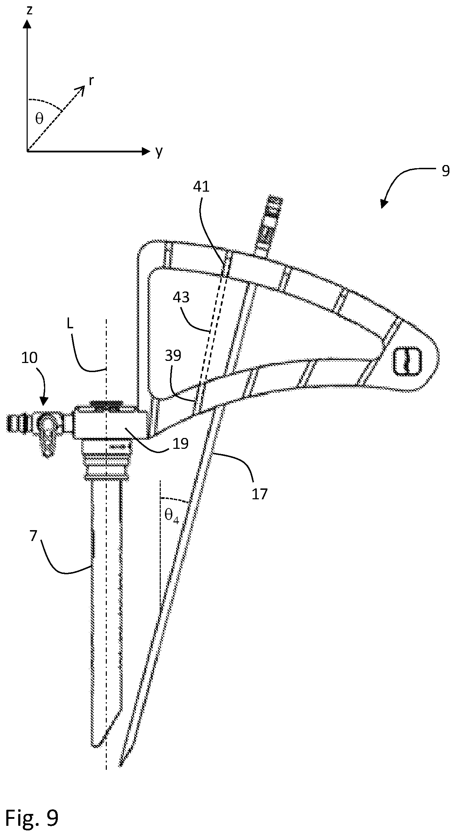

Fig. 9 zeigt ein anderes Ausführungsbeispiel eines Zielgeräts 9, das an dem Zugangstubus 7 befestigt ist. Die weniger komplexe Struktur des Zielgeräts 9 kann ggf. leichter in einem nicht-additiven Fertigungsverfahren hergestellt, beispielsweise gegossen und/oder gefräst, werden. Die insgesamt neun Hohlnadelführungen 21 weisen hier jeweils einen distalen Führungsabschnitt 39 und einen proximalen Führungsabschnitt 41 auf, wobei das Zielgerät 9 zwischen dem distalen Führungsabschnitt 39 und dem proximalen Führungsabschnitt 41 zumindest einen freien Abschnitt 43 aufweist, in dem die Hohlnadel 17 nicht geführt ist. Auch dies kann Gewicht und Material sparen, da die Hohlnadel 17 grundsätzlich nur an zwei in Längsrichtung möglichst weit beabstandeten Stellen geführt sein muss, um die räumliche Ausrichtung der Hohlnadel 17 eindeutig festzulegen. Die Länge des freien Abschnitts 43 ist für näher an der Endoskopie-Längsachse L gelegene Hohlnadelführungen 21 länger als für weiter von der Endoskopie-Längsachse L beabstandete Hohlnadelführungen 21. Die Eigenschaften der Ausführungsbeispiele gemäß Fig. 2 bis 8a,b können beliebig mit den Eigenschaften des Ausführungsbeispiels gemäß Fig. 9 kombiniert werden. In Fig. 9 ist die Hohlnadel 17 in der vierten Hohlnadelführung 21 eingesetzt, die sich entlang des Polwinkels θ₄ relativ zur Endoskopie-Längsachse L erstreckt und wie die zweite, sechste und achte Hohlnadelführung 21 zur nicht sichtbaren azimutalen Seite hin geöffnet ist. Die erste, dritte, fünfte, siebte und neunte Hohlnadelführung 21 sind jeweils zur sichtbaren azimutalen Seite hin geöffnet.

### Bezugszeichenliste:

- 1: Endoskopie-Einheit
- 3: Endoskopie-System
- 5: Endoskop
- 7: Zugangstubus
- 9: Zielgerät
- 10: Spülflüssigkeitseinlass und/oder -auslass
- 11: Sichtbereich
- 13: Okular
- 15: proximale Arbeitskanalöffnung
- 17: Hohlnadel
- 19: Befestigungselement
- 21: Hohlnadelführung
- 23: proximales Führungsende
- 25: distales Führungsende
- 27: Durchbrechung
- 29: Öffnungsschlitz
- 30: Hohlkammer
- 31: Griffhaken
- 33: Schnellspannhebel
- 35: Formschlusselement
- 37: Formschlusselement
- 39: distaler Führungsabschnitt
- 41: proximaler Führungsabschnitt
- 43: freier Abschnitt
- A: Abstand
- D: Innendurchmesser
- S: lichte Schlitzweite
- θ: Polwinkel
- φ: Azimut-Winkel

## Patentansprüche

1. Zielgerät (9) zur gezielten Führung einer Hohlnadel (17) bei einem endoskopisch-chirurgischen Eingriff, wobei das Zielgerät (9) an einer sich im Wesentlichen in einer Endoskopie-Längsachse (L) erstreckenden und einen Sichtbereich (11) definierenden Endoskopie-Einheit (1) befestigt oder befestigbar ist, wobei das Zielgerät (9) eine Mehrzahl von Hohlnadelführungen (21) definiert, wobei sich jede der Hohlnadelführungen (21) jeweils entlang eines anderen Polwinkels (θ) gegenüber der Endoskop-Längsachse (L) auf den Sichtbereich (11) zu erstreckt, **dadurch gekennzeichnet, dass**
jede der Hohlnadelführungen (21) über ihre gesamte Länge zu einer ihrer zwei azimutalen Seiten hin offen ist, und dass
das Zielgerät (9) zwischen den Hohlnadelführungen (21) azimutal durchbrochen ist.

2. Zielgerät (9) nach Anspruch 1, wobei jede Hohlnadelführung (21) jeweils ein proximales Führungsende (23) und ein distales Führungsende (25) aufweist, wobei die proximalen Führungsenden (23) der Hohlnadelführungen (21) im Wesentlichen den gleichen Abstand vom Sichtbereich (11) haben.

3. Zielgerät (9) nach Anspruch 2, wobei für jedes beliebig aus den Hohlnadelführungen (21) gewählte Paar gilt, dass sich das distale Führungsende (25) einer ersten Hohlnadelführung (21) des Paars entlang eines ersten Polwinkels (θ) erstreckt und einen ersten Abstand vom Sichtbereich (11) hat und sich das distale Führungsende (25) einer zweiten Hohlnadelführung (21) des Paars entlang eines zweiten Polwinkels (θ) erstreckt und einen zweiten Abstand vom Sichtbereich (11) hat, wobei der erste Polwinkel (θ) kleiner als der zweite Polwinkel (θ) ist und der erste Abstand kleiner als der zweite Abstand ist.

4. Zielgerät (9) nach Anspruch 2 oder 3, wobei sich die proximalen Führungsenden (23) der Hohlnadelführungen (21) proximalwärts trichterartig aufweiten und jeweils einen definierten Anschlag für die Hohlnadel (17) bilden.

5. Zielgerät (9) nach einem der Ansprüche 2 bis 4, wobei benachbarte Hohlnadelführungen (21) jeweils am proximalen Führungsende (23) und am distalen Führungsende (25) miteinander verbunden sind.

6. Zielgerät (9) nach einem der vorhergehenden Ansprüche, wobei die Hohlnadelführungen (21) abwechselnd zu unterschiedlichen ihrer zwei azimutalen Seiten hin offen sind.

7. Zielgerät (9) nach einem der vorhergehenden Ansprüche, wobei die Hohlnadelführungen (21) durch ein einstückiges Führungselement gebildet sind.

8. Zielgerät (9) nach einem der vorhergehenden Ansprüche, wobei zumindest eine der Hohlnadelführungen (21) einen distalen Führungsabschnitt (39) und einen proximalen Führungsabschnitt (41) aufweist, wobei das Zielgerät (9) zwischen dem distalen Führungsabschnitt (39) und dem proximalen Führungsabschnitt (41) zumindest einen freien Abschnitt (43) aufweist, in dem die Hohlnadel (17) nicht geführt ist.

9. Zielgerät (9) nach Anspruch 8, wobei für jedes beliebig aus den Hohlnadelführungen (21) gewählte Paar gilt, dass sich eine erste Hohlnadelführung (21) des Paars entlang eines ersten Polwinkels (θ) erstreckt und sich eine zweite Hohlnadelführung (21) des Paars entlang eines zweiten Polwinkels (θ) erstreckt, wobei der freie Abschnitt (43) der ersten Hohlnadelführung (21) länger ist als der freie Abschnitt (43) der zweiten Hohlnadelführung (21) und der erste Polwinkel (θ) kleiner als der zweite Polwinkel (θ) ist.

10. Zielgerät (9) nach einem der vorhergehenden Ansprüche, wobei für jedes beliebig aus den Hohlnadelführungen (21) gewählte Paar gilt, dass sich eine erste Hohlnadelführung (21) des Paars entlang eines ersten Polwinkels (θ) erstreckt und sich eine zweite Hohlnadelführung (21) des Paars entlang eines zweiten Polwinkels (θ) erstreckt, wobei die Länge der ersten Hohlnadelführung (21) größer ist als die Länge der zweiten Hohlnadelführung (21) und der erste Polwinkel (θ) kleiner als der zweite Polwinkel (θ) ist.

11. Zielgerät (9) nach einem der vorhergehenden Ansprüche, wobei jede der Hohlnadelführungen (21) einen Innendurchmesser (D) zur axialen Aufnahme der Hohlnadel (17) hat und auf ihrer offenen azimutalen Seite einen Öffnungsschlitz (29) mit einer lichten Schlitzbreite (S) aufweist, wobei die lichte Schlitzbreite (S) kleiner ist als der Innendurchmesser (D).

12. Zielgerät (9) nach einem der vorhergehenden Ansprüche, wobei die Hohlnadelführungen (21) meridional aufgereiht angeordnet sind.

13. Endoskopie-System (3) für einen endoskopisch-chirurgischen Eingriff, wobei das Endoskopie-System (3) aufweist:
- eine sich im Wesentlichen in einer Endoskopie-Längsachse (L) erstreckende und einen Sichtbereich (11) definierende Endoskopie-Einheit (1),
- mindestens eine Hohlnadel (17), und
- ein Zielgerät (9) nach einem der vorhergehenden Ansprüche zur gezielten Führung der mindestens einen Hohlnadel (17) entlang eines auswählbaren Polwinkels (θ) gegenüber der Endoskop-Längsachse (L) auf den Sichtbereich (11) zu, wobei das Zielgerät (9) an der Endoskopie-Einheit (1) befestigt oder befestigbar ist.

14. Endoskopie-System (3) nach Anspruch 13, wobei die Endoskopie-Einheit (1) einen sich im Wesentlichen in der Endoskopie-Längsachse (L) erstreckenden Zugangstubus (7) und ein von proximal in den Zugangstubus (7) einsetzbares Endoskop (5) aufweist.

## Claims

1. A targeting device (9) for targeted guidance of a hollow needle (17) during an endoscopic-surgical operation, wherein the targeting device (9) is fastened or fastenable to an endoscopy unit (1) which extends substantially in an endoscopy longitudinal axis (L) and defines a viewing region (11), wherein the targeting device (9) defines a plurality of hollow needle guides (21), wherein each of the hollow needle guides (21) each extends towards the viewing region (11) along a different polar angle (θ) with respect to the endoscopy longitudinal axis (L), **characterised in that** each of the hollow needle guides (21)
is open towards one of its two azimuthal sides over its entire length, and **in that**
the targeting device (9) is azimuthally broken through between the hollow needle guides (21).

2. The targeting device (9) according to claim 1, wherein each hollow needle guide (21) has a proximal guide end (23) and a distal guide end (25), wherein the proximal guide ends (23) of the hollow needle guides (21) substantially have the same distance from the viewing region (11).

3. The targeting device (9) according to claim 2, wherein for every pair which is selected arbitrarily from the hollow needle guides (21), the distal guide end (25) of a first hollow needle guide (21) of the pair extends along a first polar angle (θ) and has a first distance from the viewing region (11) and the distal guide end (25) of a second hollow needle guide (21) of the pair extends along a second polar angle (θ) and has a second distance from the viewing region (11), wherein the first polar angle (θ) is smaller than the second polar angle (θ) and the first distance is smaller than the second distance.

4. The targeting device (9) according to claim 2 or 3, wherein the proximal guide ends (23) of the hollow needle guides (21) widen proximally in a funnel-like manner and in each case form a defined stop for the hollow needle (17).

5. The targeting device (9) according to one of claims 2 to 4, wherein adjacent hollow needle guides (21) are connected to one another in each case at the proximal guide end (23) and at the distal guide end (25).

6. The targeting device (9) according to one of the preceding claims, wherein the hollow needle guides (21) are open towards different ones of their two azimuthal sides in an alternating manner.

7. The targeting device (9) according to one of the preceding claims, wherein the hollow needle guides (21) are formed by a one-piece guide element.

8. The targeting device (9) according to one of the preceding claims, wherein at least one of the hollow needle guides (21) has a distal guide portion (39) and a proximal guide portion (41), wherein the targeting device (9) between the distal guide portion (39) and the proximal guide portion (41) has at least one free portion (43), in which the hollow needle (17) is not guided.

9. The targeting device (9) according to claim 8, wherein for each pair selected arbitrarily from the hollow needle guides (21), a first hollow needle guide (21) of the pair extends along a first polar angle (θ) and a second hollow needle guide (21) of the pair extends along a second polar angle (θ), wherein the free portion (43) of the first hollow needle guide (21) is longer than the free portion (43) of the second hollow needle guide (21) and the first polar angle (θ) is smaller than the second polar angle (θ).

10. The targeting device (9) according to one of the preceding claims, wherein for each pair selected arbitrarily from the hollow needle guides (21), a first hollow needle guide (21) of the pair extends along a first polar angle (θ) and a second hollow needle guide (21) of the pair extends along a second polar angle (θ), wherein the length of the first hollow needle guide (21) is larger than the length of the second hollow needle guide (21) and the first polar angle (θ) is smaller than the second polar angle (θ).

11. The targeting device (9) according to one of the preceding claims, wherein each of the hollow needle guides (21) has an inner diameter (D) for axially receiving the hollow needle (17) and at its open azimuthal side has an opening slot (29) with a clear slot width (S), wherein the clear slot width (S) is smaller than the inner diameter (D).

12. The targeting device (9) according to one of the preceding claims, wherein the hollow needle guides (21) are arranged in a meridionally rowed manner.

13. An endoscopy system (3) for an endoscopic-surgical operation, wherein the endoscopy system (3) comprises:
- an endoscopy unit (1) which extends substantially in an endoscopy longitudinal axis (L) and defines a viewing region (11);
- at least one hollow needle (17); and
- a targeting device (9) according to one of the preceding claims for targeted guidance of the at least one hollow needle (17) towards the viewing region (11) along a selectable polar angle (θ) with respect to the endoscopy longitudinal axis (L), wherein the targeting device (9) is fastened or fastenable to the endoscopy unit (1).

14. The endoscopy system (3) according to claim 13, wherein the endoscopy unit (1) has an access tube (7) extending substantially in the endoscopy longitudinal axis (L) and an endoscope (5) which can be inserted into the access tube (7) from a proximal side.

## Revendications

1. Dispositif de ciblage (9) pour le guidage ciblé d'une aiguille creuse (17) lors d'une intervention endoscopique chirurgicale, le dispositif de ciblage (9) étant fixé ou pouvant être fixé à une unité d'endoscopie (1) s'étendant sensiblement dans un axe longitudinal d'endoscopie (L) et définissant un champ de vision (11), le dispositif de ciblage (9) définissant une pluralité de guides d'aiguille creuse (21), chacun des guides d'aiguilles creuses (21) s'étendant respectivement le long d'un autre angle polaire (θ) par rapport à l'axe longitudinal endoscopique (L) vers la champ de vision (11), **caractérisé en ce que**
chacun des guides d'aiguille creuse (21)
étant ouvert sur toute sa longueur vers l'un de ses deux côtés azimutaux, et **en ce que**
le dispositif de ciblage (9) est interrompu azimutalement entre les guides d'aiguille creuse (21).

2. Dispositif de ciblage (9) selon la revendication 1, chaque guide d'aiguille creuse (21) ayant respectivement une extrémité de guide proximale (23) et une extrémité de guide distale (25), les extrémités de guidage proximales (23) des guides d'aiguille creuse (21) présentant sensiblement la même distance par rapport à la champ de vision (11).

3. Dispositif de ciblage (9) selon la revendication 2, dans lequel il s'avère, pour chaque paire choisie parmi les guides d'aiguille creuse (21), que l'extrémité de guide distale (25) d'un premier guide d'aiguille creuse (21) de la paire s'étend le long d'un premier angle polaire (θ) et présente une première distance par rapport au champ de vision (11) et que l'extrémité de guide distale (25) d'un second guide d'aiguille creuse (21) de la paire s'étend le long d'un second angle polaire (θ) et une seconde distance par rapport au champ de vision (11), le premier angle polaire (θ) étant inférieur au second angle polaire (θ) et la première distance étant inférieure à la seconde distance.

4. Dispositif de ciblage (9) selon la revendication 2 ou 3, dans lequel les extrémités de guide proximales (23) des guides d'aiguille creuse (21) s'élargissent en forme d'entonnoir dans la direction proximale et forment chacune une butée définie pour l'aiguille creuse (17).

5. Dispositif de ciblage (9) selon l'une des revendications 2 à 4, dans lequel les guides d'aiguille creuse adjacents (21) sont respectivement reliés entre eux à l'extrémité de guide proximal (23) et à l'extrémité de guide distale (25).

6. Dispositif de ciblage (9) selon l'une des revendications précédentes, dans lequel les guides d'aiguille creuse (21) sont ouverts alternativement vers des côtés différents parmi leurs deux côtés azimutaux.

7. Dispositif de ciblage (9) selon l'une des revendications précédentes, dans lequel les guides d'aiguille creuse (21) sont formés par un élément de guidage en une seule pièce.

8. Dispositif de ciblage (9) selon l'une des revendications précédentes, dans lequel au moins un des guides d'aiguille creuse (21) présente une section de guide distale (39) et une section de guide proximale (41), le dispositif de ciblage (9) présentant entre la section de guide distale (39) et la section de guide proximale (41) au moins une section libre (43) dans laquelle l'aiguille creuse (17) n'est pas guidée.

9. Dispositif de ciblage de ciblage (9) selon la revendication 8, dans lequel il s'avère que, pour chaque paire choisie à volonté parmi les guides d'aiguille creuse (21), qu'un premier guide d'aiguille creuse (21) de la paire s'étend le long d'un premier angle polaire (θ) et qu'un second guide d'aiguille creuse (21) de la paire s'étend le long d'un second angle polaire (θ), la section libre (43) du premier guide d'aiguille creuse (21) étant plus longue que la section libre (43) du second guide d'aiguille creuse (21) et le premier angle polaire (θ) étant inférieur au second l'angle polaire (θ).

10. Dispositif de ciblage (9) selon l'une des revendications précédentes, dans lequel il s'avère que, pour chaque paire choisie à volonté parmi les guides d'aiguille creuse (21), qu'un premier guide d'aiguille creuse (21) de la paire s'étend le long d'un premier angle polaire (θ) et qu'un second guide d'aiguille creuse (21) de la paire s'étend le long d'un second angle polaire (θ), la longueur du premier guide d'aiguille creuse (21) étant supérieure à la longueur du second guide d'aiguille creuse (21) et le premier angle polaire (θ) étant inférieur au second angle polaire (θ).

11. Dispositif de ciblage (9) selon l'une des revendications précédentes, chacun des guides d'aiguille creuse (21) a un diamètre intérieur (D) destiné à recevoir axialement l'aiguille creuse (17) et présente sur son côté azimutal ouvert une fente d'ouverture (29) avec une largeur de fente libre (S), la largeur de fente libre (S) étant inférieure au diamètre intérieur (D).

12. Dispositif de ciblage (9) selon l'une des revendications précédentes, dans lequel les guides d'aiguille creuse (21) sont disposés en rangs méridiens.

13. Système d'endoscopie (3) pour intervention endoscopique et chirurgicale, le système d'endoscopie (3) présentant:
- une unité d'endoscopie qui s'étend sensiblement dans un axe longitudinal d'endoscopie (L) et qui définit un champ de vision (11) (1),
- au moins une aiguille creuse (17), et
- un dispositif de ciblage (9) selon l'une des revendications précédentes pour guider de manière ciblée l'au moins une aiguille creuse (17) le long d'un angle polaire sélectionnable (θ) par rapport à l'axe longitudinal endoscopique (L) vers le champ de vision (11), le dispositif de ciblage (9) étant fixé ou pouvant être fixé à l'unité d'endoscopie (1).

14. Système d'endoscopie (3) selon la revendication 13, dans lequel l'unité d'endoscopie (1) comporte un tube d'accès (7) qui s'étend sensiblement dans l'axe longitudinal d'endoscopie (L) et un endoscope (5) qui peut être inséré proximalement dans le tube d'accès (7).
